# EUROPÄISCHE PATENTANMELDUNG

(11) **EP 4 389 869 A1**
(43) Veröffentlichungstag der Anmeldung: **26.06.2024**
(21) Anmeldenummer: 22216054.1
(22) Anmeldetag: 22.12.2022
(51) Int. Cl.: C12M 1/00, C12M 3/00

(54) **PHOTOBIOREAKTOR ZUR PRODUKTION VON PHOTOSYNTHESE BETREIBENDEN ORGANISMEN**

(71) Anmelder: AT-Solid GmbH, 37115 Duderstadt (DE)
(72) Erfinder: HAVERS, Martin, 58708 Menden (DE); PETRASCH, Hans, 55425 Waldalgesheim (DE)
(74) Vertreter: Neumann Müller Oberwalleney Patentanwälte PartG mbB

(57) **Zusammenfassung**

Photobioreaktor zur Produktion von Photosynthese betreibenden Organismen mit mehreren zueinander beabstandeten Behältern zur Aufnahme eines Nährstoffmediums.

## Beschreibung

Die Anmeldung betrifft einen Photobioreaktor zur Produktion von Photosynthese betreibenden Organismen mit mehreren zueinander beabstandeten Behältern zur Aufnahme eines Nährstoffmediums.

In Photobioreaktoren können mit Licht, Kohlendioxid, Nährstoffen sowie Wasser und Salzen Mikroorganismen vermehrt werden. Die Mikroorganismen bzw. deren Bestandteile dienen beispielsweise zur Herstellung von Kraftstoffen, Nahrungsmitteln, Nahrungsmittelzusatzstoffen, Enzymen, Ölen, Proteinen, pharmakologischen Wirkstoffen, chemischen Produkten und Kunststoffen oder werden in Aquakulturen, in Tierfutter, der Umwelttechnik und für Kosmetik und Pflege genutzt. Ein Photobioreaktor ist eine Anlage zur Produktion der Mikroorganismen in einer künstlichen technischen Umgebung. Ein grundlegendes Problem besteht darin, dass das Licht nicht mehr alle Bereiche des Bioreaktors erreichen kann, wenn sich die phototrophen Mikroorganismenkulturen vermehren und die Flüssigkeit durch den höheren Chlorophyllanteil grün wird. Eine zu geringe Lichtstärke erreicht das Innere des Bioreaktors und das Wachstum der Biomasse verzögert sich.

Die Druckschrift EP 3 041 924 A1 offenbart eine Vorrichtung zur Gewinnung von Phytoplankton (Mikroalgen), bei der in einem Gehäuse eine Nährstofflösung und mehrere vertikal ausgerichtete und in horizontalem Abstand zueinander angeordnete Platten vorhanden sind, die alternierend entweder am Boden oder an der Oberseite des Gehäuses befestigt sind und sich nicht bis zur gegenüberliegenden Wand erstrecken, um eine vertikal mäanderförmige Strömung auszubilden sowie die Nährstofflösung über eine Pumpe umgewälzt wird, wobei an der im Bereich der Befestigung befindlichen Stirnfläche der Platte Beleuchtungsmittel angebracht sind und die Platte aus transparentem Festmaterial besteht, in das Lichtstreuende Partikel derart eingebettet sind, dass die Dichte des Lichtaustrittes über die Oberfläche der Platte in etwa konstant ist.

Die mäanderförmig umströmten Platten sollen das eingekoppelte Licht in den Photobioreaktor abstrahlen. Durch die Platten werden jedoch auch zahlreiche Winkel und Ecken gebildet, in denen kaum Strömung herrscht, was zu nachteiligem Anhaften der Mikroorganismen an den Platten und an den Wänden des Photobioreaktors führt. Eine Reinigung des Photobioreaktors wird durch die Platten erschwert.

Eine Aufgabe kann darin bestehen, die Nachteile des zuvor beschriebenen Photobioreaktors zu überwinden.

Die Aufgabe wird durch einen Photobioreaktor gemäß Anspruch 1 gelöst. In den Unteransprüchen sind vorteilhafte Ausführungsformen angegeben.

Der Photobioreaktor zur Produktion von Photosynthese betreibenden Organismen weist mehrere zueinander beabstandete Behältern zur Aufnahme eines Nährstoffmediums auf, wobei den Behältern eine Beleuchtung zugeordnet ist und wobei die Behälter untereinander fluidisch verbunden sind. Die Behälter sind aus jeweils mindestens fünf Wänden gebildet, wobei die Wände ein quaderförmiges Innenvolumen definieren. Die Behälter weisen jeweils mindestens eine Gaszuführung im Bereich einer Bodenwand auf.

Ein Vorteil des Photobioreaktors besteht darin, dass das quaderförmige Innenvolumen der Behälter besonders günstig mechanisch zu reinigen ist, so dass beispielsweise eine maschinelle bzw. automatisierte Reinigung der Behälter erfolgen kann. Die Gaszuführung im Bereich der Bodenwand erlaubt weiterhin vorteilhaft eine Durchmischung des Nährstoffmediums und der Organismen in dem Behälter, wobei durch aufsteigendes Gas im Bereich der Gaszuführung eine Strömung gegen die Schwerkraftrichtung zu einer Oberfläche der Nährstofflösung erzeugt wird, die außerhalb des Bereichs der Gaszuführung wieder in Schwerkraftrichtung zurückführt. Der Behälterinhalt wird somit laufend umgewälzt, sodass die Organismen sich wechselnd im Inneren des Behälters und nahe den Wänden bewegen. Durch die Umwälzung erhalten die Organismen auch bei fortgeschrittenem Wachstum ausreichend Licht. Ein weiterer Vorteil der Umwälzung besteht darin, dass ein Anhaften der Mikroorganismen an den Wänden des Photobioreaktors vermindert wird.

Das quaderförmige Innenvolumen des Behälters ist mit Ausnahme von Fluidleitungen monolithisch, also insbesondere frei von Zwischenwänden, Stegen oder ähnlichen, die Strömung beeinflussenden Bauteilen. Somit ist das quaderförmige Innenvolumen mit Ausnahme der optional vorhandenen Fluidleitungen ausschließlich mit fluiden Medien und den darin wachsenden Photosynthese betreibenden Organismen gefüllt. Die fluiden Medien umfassen insbesondere das Nährstoffmedium und Gase. Der Fachmann erkennt, dass der Behälter in der Regel nicht vollständig gefüllt sein wird, sondern dass das quaderförmige Innenvolumen oberhalb des flüssigen Nährstoffmediums mit Gas, insbesondere mit Luft gefüllt ist. Ein abnehmbarer Deckel kann den Behälter als sechste Wand nach oben verschließen. Das quaderförmige Innenvolumen der Behälter beträgt beispielsweise zwischen 200 Liter und 10000 Liter, wobei eine kürzeste Kante des quaderförmigen Innenvolumens mindesten zehn Zentimeter lang ist.

Gemäß einer Ausführungsform weisen die Behälter jeweils mindestens ein Ablassventil zur Entnahme des Nährstoffmediums auf. Die Ablassventile können fluidisch mit einer Sammelleitung verbunden sein, die beispielsweise mit einer Verwertungsanlage verbunden sein kann, in der das mit den Photosynthese betreibenden Organismen angereicherte Nährstoffmedium verarbeitet wird. Dabei kann die Konzentration der Photosynthese betreibenden Organismen beispielsweise mittels Zentrifugierens erhöht werden. Des Weiteren können die Photosynthese betreibenden Organismen durch Filtrierung von der Nährstofflösung getrennt werden. Die Photosynthese betreibenden Organismen können anschließend getrocknet und beispielsweise zu Pulver vermahlen werden. Die Nährstofflösung kann nach der Filtration wiederaufbereitet und erneut den Behältern zur Produktion der Photosynthese betreibenden Organismen zugeführt werden. Alternativ kann die Sammelleitung zur Entsorgung des Inhalts einzelner Behälter oder des gesamten Photobioreaktors umgeleitet werden, falls die Produktion verworfen werden soll.

Gemäß einer weiteren Ausführungsform sind die Behälter jeweils mittels einer Bypassleitung überbrückbar. Dadurch kann vorteilhaft ein einzelner oder mehrere Behälter des Photobioreaktors aus der ansonsten weiter laufenden Produktion ausgekoppelt werden, beispielsweise um Wartungs- oder Reinigungsarbeiten an dem Behälter durchzuführen, der dazu auch bei Bedarf über das Ablassventil entleer werden kann.

Gemäß einer weiteren Ausführungsform sind die Behälter in Reihe fluidisch verbunden, wobei eine Rücklaufleitung eine Zirkulation des Nährstoffmediums ermöglicht. Zur Zirkulierung des Nährstoffmediums kann mindestens eine Pumpe vorgesehen sein, die beispielweise im Bereich der Rücklaufleitung vorgesehen ist. Die Pumpe kann insbesondere eine Exzenterschneckenpumpe (progressing cavity pump) sein, in deren Hohlräumen mit konstanter Form die Nährstofflösung bei der Förderung vorteilhaft nicht komprimiert wird, was sich positiv auf die Photosynthese betreibenden Organismen darin auswirkt.

Gemäß einer weiteren Ausführungsform sind Sensoren zur Ermittlung mindestens eines der Parameter Salzgehalt, Temperatur, Kohlendioxid-Gehalt, Trübung, Sauerstoffgehalt, optische Dichte, elektrische Leitfähigkeit, pH-Wert, Ammoniakgehalt (NH3), Nitrat/Nitrit Gehalt (NO3), Druck, Füllstand der Nährstofflösung vorgesehen sind, wobei die Sensoren insbesondere in der Rücklaufleitung angeordnet sind.

Gemäß einer weiteren Ausführungsform weist die Gaszuführung mindestens eine Düse auf, wobei die Düse im einfachsten Fall eine Bohrung in einer Gasführenden Leitung, Rohr oder Schlauch, sein kann. Insbesondere ist eine Vielzahl von Düsen vorgesehen. Der Gasdurchsatz ist beispielsweise über ein Regelventil steuerbar. Die Düsen können bezüglich mindestens eines der Parameter Gasdurchsatz und Düsengeometrie regelbar sein.

Gemäß einer weiteren Ausführungsform ist die Beleuchtung eine LED-Beleuchtung, bei der mindestens einer der Parameter Bestrahlungsintensität, Dimmung, Lichtwellenspektrum und Flackerfrequenz einstellbar ist. Die Photosynthese betreibenden Organismen nutzen den Prozess der Photosynthese, um aus Licht und Kohlendioxid ihre eigene Biomasse aufzubauen. Zu diesen Organismen zählen beispielsweise Pflanzen, Moose, Makroalgen, Mikroalgen, Cyanobakterien und Purpurbakterien. Der Photobioreaktor dient zur kontrollierten Bereitstellung eines Lebensraums, der für den jeweiligen Organismus die optimalen Lebensbedingungen bietet. Damit ermöglicht ein Photobioreaktor deutlich höhere Wachstumsraten und Reinheiten, als es in einer natürlichen oder naturähnlichen Umgebung der Fall wäre.

Nachfolgend wird die Erfindung mit Bezug auf die beiliegenden Zeichnungen näher erläutert.

Es zeigt
- Figur 1: eine Ausführungsform eines Photobioreaktors;
- Figur 2: eine weitere Ausführungsform des Photobioreaktors;
- Figur 3: ein Detail des Photobioreaktors gemäß Figur 1 ;
- Figur 4: ein Detail einer weiteren Ausführungsform des Photobioreaktors;

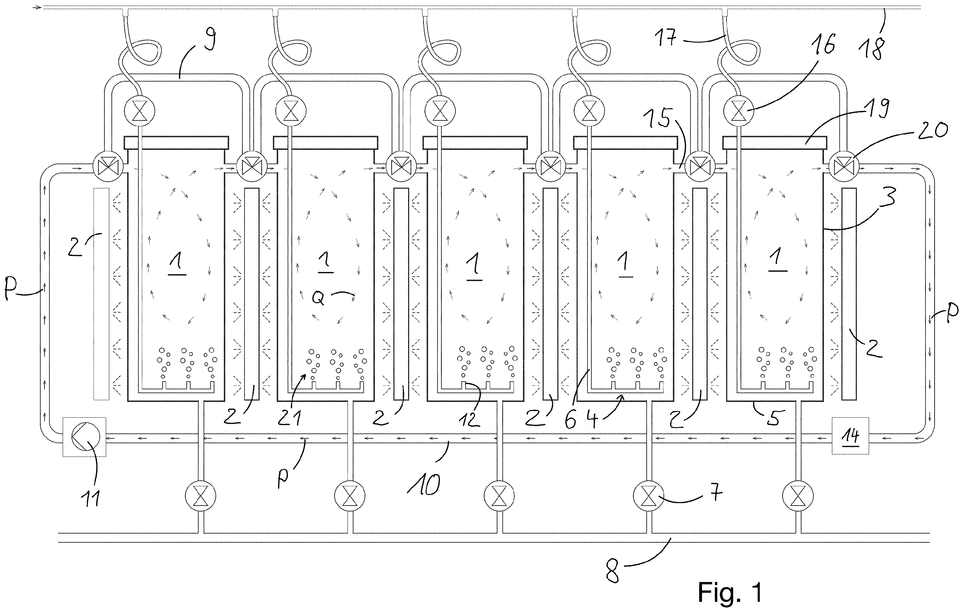

In der Figur 1 ist eine Ausführungsform eines Photobioreaktors schematisch dargestellt. Der Photobioreaktor zur Produktion von Photosynthese betreibenden Organismen weist mehreren zueinander beabstandete Behälter 1 zur Aufnahme eines Nährstoffmediums auf. Den Behältern 1 ist eine Beleuchtung 2 zugeordnet, wobei ein Abstand zwischen den Behältern 1 so bemessen ist, dass in einem Zwischenraum zwischen den Behältern 1 jeweils die Beleuchtung 2 bildende ebene, flächige Lichtmodull auf Basis von LED angeordnet werden können. Dabei bilden die Lichtmodule einen sogenannten Lichtvorhang, der sein Licht in beiden Richtungen abstrahlt. Die LEDs lassen sich beispielsweise in der Zeitphase, Intensität und in der spektralen Verteilung des Lichts individuell steuern. Den jeweils endseitig angeordneten Behälter 1 sind jeweils ein sogenannter Endvorhang der Beleuchtung zugeordnet, der Licht nur in einer Richtung abgibt.

Die Behälter 1 sind aus jeweils mindestens fünf Wänden 3, 5 gebildet. Die vier Seitenwände 3 und die Bodenwand 5 bilden ein quaderförmiges Innenvolumen, wobei das quaderförmige Innenvolumen mit Ausnahme von Fluidleitungen 6, die durch das Innenvolumen verlaufen, monolithisch ist. Das quaderförmige Innenvolumen ist somit mit Ausnahme der Fluidleitungen 6 ausschließlich mit fluiden Medien und den Photosynthese betreibenden Organismen gefüllt, wobei die fluiden Medien insbesondere das Nährstoffmedium und Gase umfassen. Die Behälter 1 weisen jeweils mindestens eine Gaszuführung 4 im Bereich der Bodenwand 5 auf, die über die Fluidleitung 6 mit Gas versorgt wird. Die Gaszuführung 4 kann mehrere Düsen 12 aufweisen, die bezüglich eines Gasdurchsatzes regelbar sind. Die Regelung des Gasdurchsatzes kann über Ventile 16 in einer Gaszuführleitung 17 erfolgen, die eine Gasversorgungsleitung 18 mit der Fluidleitung verbunden ist. Ein abnehmbarer Deckel 19 kann die Behälter 1 nach oben verschließen, wobei die Gaszuführung durch den Deckel 19 erfolgt.

Die Behälter 1 sind untereinander fluidisch über Zu- und Ablaufleitungen 15 verbunden. Die einzelnen Behälter 1 können sowohl in einer horizontalen Ebene als auch leicht gestuft aufgestellt werden, um ein Absenken des Nährstoffmediums im letzten Behälter 1 zu verhindern. Die Behälter 1 sind in Reihe fluidisch verbunden, wobei eine Rücklaufleitung 10 eine Zirkulation des Nährstoffmediums ermöglicht. Mindestens eine Pumpe 11 ist zur Zirkulation des Nährstoffmediums im Bereich der Rücklaufleitung 10 vorgesehen, insbesondere eine drehzahlvariable Pumpe 11, die geeignet ist, die Mikroorganismen zu fördern, ohne diese zu schädigen. Die Pfeile P geben eine Fließrichtung des Nährstoffmediums durch die Rücklaufleitung an. Die die endseitigen Behälter 1 verbindende Rücklaufleitung 10 hat nicht nur die Aufgabe, die Behälter 1 miteinander zu verbinden, sondern in dieser Rücklaufleitung 10 können Sensoren 14 angeordnet sein. Beispielsweise dienen die Sensoren 14 zur Ermittlung mindestens eines der Parameter Salzgehalt, Temperatur, Kohlendioxid-Gehalt, Trübung, Sauerstoffgehalt, optische Dichte, elektrische Leitfähigkeit, pH-Wert, Ammoniakgehalt NH3, Nitrat/Nitrit Gehalt NO3, Druck, Füllstand der Nährstofflösung. Schematisch dargestellte Gasblasen 21 stellen den Austritt von Gas aus der Gaszuführung 4 dar. Dieser Gasaustritt sorgt für eine Verwirbelung des Nährstoffmediums in dem Behälter 1, der schematisch durch die Pfeile Q dargestellt ist.

Die Behälter 1 weisen jeweils ein Ablassventil 7 zur Entnahme des Nährstoffmediums auf, das fluidisch mit einer Sammelleitung 8 verbunden ist. Die Sammelleitung 8 kann mit einer Verwertungsanlage (nicht dargestellt) verbunden sein, in der die Photosynthese betreibenden Organismen geerntet werden. Die Behälter 1 können jeweils mittels einer Bypassleitung 9 überbrückbar sein, wobei die Nährstofflösung über Wechselventile 20 in die Bypassleitung 9 umgeleitet werden kann. Alternativ kann die Bypassleitung 9 bei Bedarf an einem oder mehreren Behältern 1 installiert und deinstalliert werden. Die Ablassventile 7 in der Bodenwand 5 der Behälter 1 können auch genutzt werden, über diese Wasser zuzuführen, was die Durchmischung der einzelnen Behälter 1 verbessert. Die einzelnen Behälter 1 des Photobioreaktors können während des Betriebs stillgelegt und herausgenommen und wieder eingebaut werden können. Erforderlich kann dies beispielsweise sein, wenn in einem Behälter 1 eine Wartungsmaßnahme oder Reinigung erforderlich ist.

Der so aus den Behältern 1 zusammengestellte Photobioreaktor kann mit weiteren Photobioreaktoren zu Photobioreaktor-Gruppen kombinieren. Diesen Photobioreaktor-Gruppen nachgeschaltet kann eine zentrale Verwertungsanlage (nicht dargestellt) sein, die beispielsweise zur Ernte, Trocknung und Verpackung der Photosynthese betreibenden Organismen dient. Dort wird das umgebende Nährstoffmedium entzogen, abgeschieden und dem Photobiorektor nach einem Reinigungs- und Filterprozess wieder zugeführt. Eine Steuerung der Photobioreaktor-Gruppen erfolgt beispielsweise vollautomatisch durch eine zentrale Steuereinheit (nicht dargestellt). Informationen der Sensoren 14 der Photobioreaktoren werden dort ausgewertet und durch einen Algorithmus ausgewertet, auf dessen Basis die Steuerung erfolgt. Dabei kann der Betriebszustand der einzelnen Photobioreaktoren unterschiedlich sein. Weiterhin werden verschiedene Störungen und Alarmzustände erfasst und die entsprechenden Gegenmaßnahmen können automatisiert eingeleitet werden.

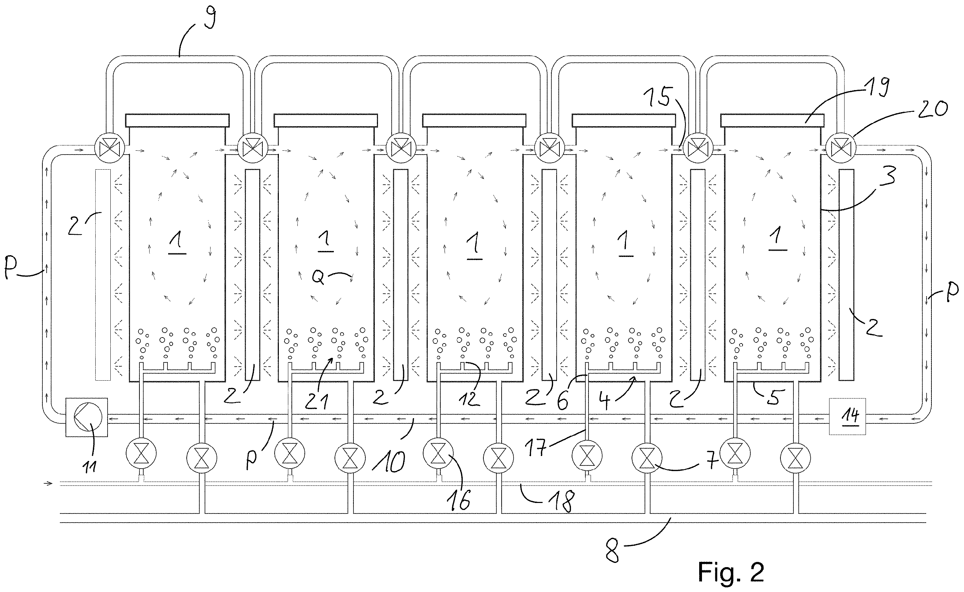

In der Figur 2 ist eine weitere Ausführungsform des Photobioreaktors schematisch dargestellt. Der Photobioreaktor zur Produktion von Photosynthese betreibenden Organismen unterscheidet sich von dem in Figur 1 dargestellten Photobioreaktor dadurch, dass die Gaszuführung 4 im Bereich der Bodenwand 5 auf über eine Fluidleitung 6 mit Gas versorgt wird, die durch die Bodenwand 5 verläuft und nicht durch den Deckel 19. Die Ventile 16 zur Regelung des Gasdurchsatzes sind in der Gaszuführleitung 17 angeordnet, die die Gasversorgungsleitung 18 mit der Fluidleitung 6 verbindet. Die übrigen Merkmale stimmen mit der Ausführungsform der Figur 1 überein.

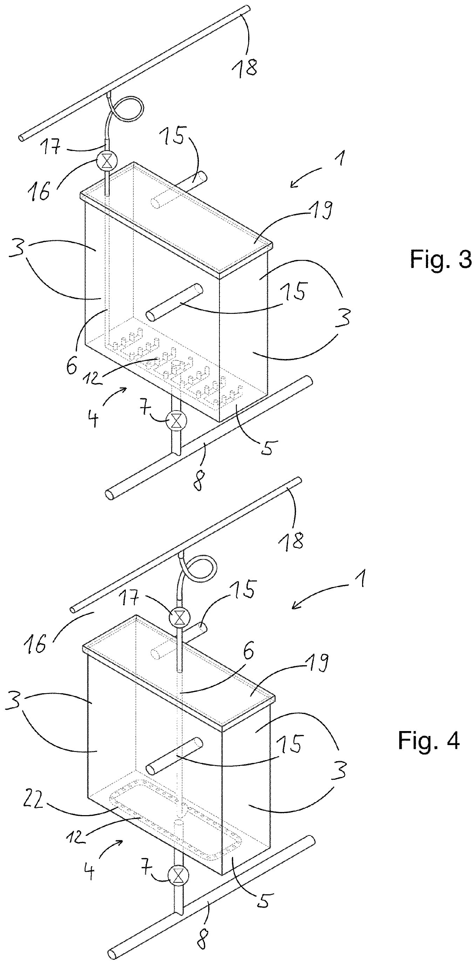

In der Figur 3 ist ein Detail des Photobioreaktors gemäß Figur 1 in einer perspektivischen Ansicht dargestellt. Die Zu- und Ablaufleitungen 15 sind versetzt zueinander in den Seitenwänden 3 des Behälters 1 angeordnet. Die Gaszuführung 4 erfolgt durch den Deckel 19, nahe einer Ecke des Behälters 1. Die Düsen 12 sind im Bereich der Bodenwand 5 des Behälters 1 angeordnet und in Richtung des Deckels 19 gerichtet.

In der Figur 4 ist ein Detail einer weiteren Ausführungsform des Photobioreaktors in einer perspektivischen Ansicht dargestellt. Der Behälter 1 unterscheidet sich von dem in Figur 3 dargestellten Behälter 1 dadurch, dass die Gaszuführung 4 eine Rohrleitung 22, beispielsweise einen Schlauch, aufweist. In der Rohrleitung 22 können die Düsen 12 in Form von Bohrungen vorgesehen sein. Die Gaszuführung 4 erfolgt etwa mittig durch den Deckel 19. Das quaderförmige Innenvolumen des Behälters 1 beträgt zwischen 200 Liter und 10000 Liter und eine kürzeste Kante des quaderförmigen Innenvolumens ist mindesten zehn Zentimeter lang.

### Bezugszeichenliste

- 1: Behälter
- 2: Beleuchtung
- 3: Wand, Seitenwand
- 4: Gaszuführung
- 5: Wand, Bodenwand
- 6: Fluidleitung
- 7: Ablassventil
- 8: Sammelleitung
- 9: Bypassleitung
- 10: Rücklaufleitung
- 11: Pumpe
- 12: Düse
- 14: Sensoren
- 15: Zu- und Ablaufleitungen
- 16: Ventile
- 17: Gaszuführleitung
- 18: Gasversorgungsleitung
- 19: Deckel
- 20: Wechselventile
- 21: Gasblasen
- 22: Rohrleitung
- P: Pfeile
- Q: Pfeile

## Patentansprüche

1. Photobioreaktor zur Produktion von Photosynthese betreibenden Organismen mit mehreren zueinander beabstandeten Behältern (1) zur Aufnahme eines Nährstoffmediums,
wobei den Behältern (1) eine Beleuchtung (2) zugeordnet ist,
wobei die Behälter (1) untereinander fluidisch verbunden sind,
wobei die Behälter (1) aus jeweils mindestens fünf Wänden (3, 5) gebildet sind und wobei die Wände (3) ein quaderförmiges Innenvolumen definieren, wobei die Behälter (1) jeweils mindestens eine Gaszuführung (4) im Bereich einer Bodenwand (5) aufweisen.

2. Photobioreaktor nach Anspruch 1, **dadurch gekennzeichnet, dass** das quaderförmige Innenvolumen mit Ausnahme von Fluidleitungen (6) monolithisch ist.

3. Photobioreaktor nach einem der vorhergehenden Ansprüche, **dadurch gekennzeichnet, dass** das quaderförmige Innenvolumen mit Ausnahme von Fluidleitungen (6) ausschließlich mit fluiden Medien und den Photosynthese betreibenden Organismen gefüllt ist, wobei die fluiden Medien insbesondere das Nährstoffmedium und Gase umfassen.

4. Photobioreaktor nach einem der vorhergehenden Ansprüche, **dadurch gekennzeichnet, dass** die Behälter (1) jeweils mindestens ein Ablassventil (7) zur Entnahme des Nährstoffmediums aufweisen.

5. Photobioreaktor nach Anspruch 4, **dadurch gekennzeichnet, dass** die Ablassventile (7) fluidisch mit einer Sammelleitung (8) verbunden sind.

6. Photobioreaktor nach Anspruch 5, **dadurch gekennzeichnet, dass** die Sammelleitung (8) mit einer Verwertungsanlage verbunden ist.

7. Photobioreaktor nach einem der vorhergehenden Ansprüche, **dadurch gekennzeichnet, dass** die Behälter (1) jeweils mittels einer Bypassleitung (9) überbrückbar sind.

8. Photobioreaktor nach einem der vorhergehenden Ansprüche, **dadurch gekennzeichnet, dass** die Behälter (1) in Reihe fluidisch verbunden sind, wobei eine Rücklaufleitung (10) eine Zirkulation des Nährstoffmediums ermöglicht.

9. Photobioreaktor nach Anspruch 8, **dadurch gekennzeichnet, dass** mindestens eine Pumpe (11) zur Zirkulation des Nährstoffmediums im Bereich der Rücklaufleitung (10) vorgesehen ist.

10. Photobioreaktor nach einem der vorhergehenden Ansprüche, **dadurch gekennzeichnet, dass** die Gaszuführung (4) mindestens eine Düse (12) aufweist, wobei die Düse bezüglich mindestens eines der Parameter Gasdurchsatz und Düsengeometrie regelbar ist.

11. Photobioreaktor nach einem der Ansprüche 8 bis 10, **dadurch gekennzeichnet, dass** Sensoren (14) zur Ermittlung mindestens eines der Parameter Salzgehalt, Temperatur, Kohlendioxid-Gehalt, Trübung, Sauerstoffgehalt, optische Dichte, elektrische Leitfähigkeit, pH-Wert, Ammoniakgehalt (NH3), Nitrat/Nitrit Gehalt (NO3), Druck, Füllstand der Nährstofflösung vorgesehen sind, wobei die Sensoren insbesondere in der Rücklaufleitung (10) angeordnet sind.

12. Photobioreaktor nach einem der vorhergehenden Ansprüche, **dadurch gekennzeichnet, dass** die Beleuchtung (2) eine LED-Beleuchtung ist, bei der mindestens einer der Parameter Bestrahlungsintensität, Dimmung, Lichtwellenspektrum und Flackerfrequenz einstellbar ist.

13. Photobioreaktor nach einem der vorhergehenden Ansprüche, **dadurch gekennzeichnet, dass** das quaderförmige Innenvolumen der Behälter (1) zwischen 200 Liter und 10000 Liter beträgt.

14. Photobioreaktor nach einem der vorhergehenden Ansprüche, **dadurch gekennzeichnet, dass** eine kürzeste Kante des quaderförmigen Innenvolumens mindesten zehn Zentimeter lang ist.
